# EUROPEAN PATENT SPECIFICATION

(11) **EP 1 991 202 B1**
(45) Date of publication and mention of the grant of the patent: **18.11.2009**
(21) Application number: 04801708.1
(22) Date of filing: 02.12.2004
(51) Int. Cl.: A61K 9/14, A61K 9/72, A61M 15/00, A61J 1/00, A61K 31/46, A61P 11/06, A61P 11/08

(54) **METHOD FOR ADMINISTRATION TIOTROPIUM**
VERFAHREN ZUR VERABREICHUNG VON TIOTROPIUM
PROCEDE D'ADMINISTRATION DE TIOTROPIUM

(30) Priority: 03.12.2003 SE 0303270
(43) Date of publication of application: 19.11.2008
(73) Proprietor: Boehringer Ingelheim International GmbH, 55216 Ingelheim am Rhein (DE)
(72) Inventor: NILSSON, Thomas, S-647 32 Mariefred (SE); MYRMAN, Mattias, S-64551 Strängnäs (SE); CALANDER, Sven, S-645 32 Strängnäs (SE); NIEMI, Alf, S-645 50 Strängnäs (SE)
(74) Representative: Eckhardt, Conrad
(86) International application number: PCT/SE2004/001791
(87) International publication number: WO 2005/053645

(56) References cited:
- WO-A1-01/27210
- WO-A1-03/084502
- WO-A1-03/086515
- WO-A1-03/086516
- WO-A1-03/086517
- WO-A2-00/74754
- US-A1- 2003 136 405

## Description

### TECHNICAL FIELD

The present invention relates to administration of bronchodilating asthma medicaments by an oral inhalation route, and more particularly doses of an anticholinergic agent are packaged to fit a new method of aerosolizing and delivering a selected dose of dry powder medicament in a single inhalation.

### BACKGROUND

Asthma and chronic obstructive pulmonary disease (COPD) affect more than 30 million people in the United States. More than 100,000 deaths each year are attributable to these conditions. Obstruction to airflow through the lungs is the characteristic feature in each of these airway diseases, and the medications utilized in treatment are often similar.

Up to 5% of the US population suffers from asthma, a respiratory condition characterized by airway inflammation, airway obstruction (at least partially reversible), and airway hyper-responsiveness to such stimuli as environmental allergens, viral respiratory-tract infections, irritants, drugs, food additives, exercise, and cold air. The major underlying pathology in asthma is airway inflammation. Inflammatory cell -- eosinophils, CD4+ lymphocytes, macrophages, and mast cells -- release a broad range of mediators, including interleukins, leukotrienes, histamine, granulocyte-colony-stimulating factor, and platelet aggregating factor. These mediators are responsible for the bronchial hyper-reactivity, bronchoconstriction, mucus secretion, and sloughing of endothelial cells.

Chronic obstructive pulmonary disease (COPD) is a widespread chronic lung disorder encompassing chronic bronchitis and emphysema. The causes of COPD are not fully understood. Experience shows that the most important cause of chronic bronchitis and emphysema is cigarette smoking. Air pollution and occupational exposures may also play a role, especially when with cigarette smoking. Heredity also causes some emphysema cases, due to alpha1 anti-trypsin deficiency.

Administration of asthma drugs by an oral inhalation route is very much in focus today, because of advantages offered like rapid and predictable onset of action, cost effectiveness and high level of comfort for the user. Dry powder inhalers (DPI) are especially interesting as an administration tool, compared to other inhalers, because of the flexibility they offer in terms of nominal dose range, i.e. the amount of active substance that can be administered in a single inhalation. So far, though, most development efforts have been directed towards producing effective drugs and formulations for specific abnormal conditions and not so much towards developing methods of administration.

When inhaling a dose of dry medication powder it is important to obtain by mass a high fine particle fraction (FPF) of particles with an aerodynamic size preferably less than 5 µm in the inspiration air. The majority of larger particles does not follow the stream of air into the many bifurcations of the airways, but get stuck in the throat and upper airways. It is not uncommon for prior art inhalers to have an efficacy of 10 - 20 % only, i.e. only 10 - 20 % of the metered dose by mass is actually delivered as particles with an aerodynamic size less than 5 µm. Since most drugs may have undesirable side effects, e.g. steroids delivered to the system, it is important to keep the dosage to the user as exact as possible and to design the delivery system, e.g. an inhaler, such that the efficacy becomes much higher than 10 - 20 %, thereby reducing the required amount of drug in the dose.

In search of methods and devices for improving dose efficacy and reducing the dosages necessary for adequate control of symptoms and respiratory disorders, some developments are to be noted. For instance, in an article in Journal of Aerosol Medicine, Volume 12, Supplement 1, 1999, pp. 33-39, the authors Pavia and Moonen report clinical studies comparing therapy efficacy of a "soft mist inhaler". Respimat^{®} from Boehringer Ingelheim KG with that of a metered dose inhaler (MDI). The studies show that the Respimat^{®} gives at least the same therapeutic bronchodilating effect as the MDI but using only half or less of the dosage in the MDI. The Respimat^{®} produces a slow-moving cloud of medicament droplets with a high fine particle fraction in a prolonged dose delivery occuring during about one second, which reduces the deposition in the oropharynx and raises the topical delivery to the correct site of action in the lung. The challenge of developing inhalers capable of producing a delivered dose with a high fine particle fraction in a prolonged dose delivery is discussed in another article in Journal of Aerosol Medicine, Volume 12, Supplement 1, 1999, pp. 3-8, by the author Ganderton.

The Respimat^{®} inhaler is a step in the right direction of cutting back the quantities of active ingredients in the doses by implementing a big increase in efficacy in the delivered dosage by adopting a prolonged dose delivery, which so far has been practically unknown in prior art.

Bronchodilating medicaments such as short-acting beta2-agonists have been used for many years in control of asthma and particularly as rescue medicines, administered as needed. Salbutamol, for instance, has very fast onset but short duration and may be administered, preferably by inhalation, several times per day in order to control attacks of dyspnoea, such that a puff of the drug provides immediate relief. Salmeterol and formoterol, both long-acting beta2-agonists, are bronchodilators, which have been used with great success for more than 20 years in the treatment of asthma. Formoterol, but not salmeterol, may be used as a rescue medicine for a quick relief of symptoms during an asthma attack. However, none of the beta2-agonists have any significant effect on underlying inflammation of the bronchi. Besides the already well-known adverse side effects of long-acting beta2-agonists (LABAs) a recent study in the US reports statistically positive evidence that salmeterol may be at the root of premature deaths caused by an acute asthma attack among salmeterol users with respiratory disorders. This is especially pronounced in the Afro-American population, which has induced FDA to issue warning messages to users of salmeterol. It is too early to say if other LABAs are afflicted with this problem. Apparently, at this time no evidence points in this very disturbing direction for short-acting beta2-agonists. However, on balance, the positive effects of a controlled treatment using LABAs and especially formoterol with its fast onset, outweigh the adverse effects. But the reported problems emphasize the need for reducing the delivered dosages of LABAs to a minimum and also look for alternative medicaments.

Anticholinergic agents, e.g. ipratropium, oxitropium and tiotropium, especially ipratropium bromide and tiotropium bromide, are also effective bronchodilators. Anticholinergic agents have a relatively fast onset and long duration of action, especially tiotropium bromide, which may be active for up to 24 hours. However, beta2-agonists and anticholinergic agents act in different ways in widening of the bronchi. Beta2-agonists help reduce contraction of the bronchial smooth muscle by stimulating the beta2-receptors, whereas an anticholinergic agent reduces vagal cholinergic tone of the smooth muscle, which is the main reversible component of COPD. Anticholinergic agents have been shown to cause quite insignificant side effects in clinical testing, dryness of mouth and constipation are perhaps the most common symptoms. Because it is often very difficult to diagnose asthma and COPD correctly and since both disorders may co-exist, it is advantageous to treat patients suffering temporary or continuous bronchial obstruction resulting in dyspnoea with a small but efficient dose of a long-acting anticholinergic agent, preferably tiotropium bromide, because of the small adverse side effects compared to LABAs.

However, selecting tiotropium as the preferred anticholinergic agent presents technical problems not found in other anticholinergic agents regarding administration methods that safeguards acceptable performance in terms of dose efficacy. Tiotropium bromide is marketed under the proprietary name of Spiriva^{®} by Boehringer Ingelheim KG and is used with a state-of-the-art DPI, the HandiHaler^{®}, as an administration tool. Retention in the inhaler tends to be a problem and the fine particle fraction in the delivered dose is generally very poor.

Thus, there is a need for improvements regarding methods of treating respiratory disorders using metered dry powder doses of a selected anticholinergic agent for administration by inhalation.

### BRIEF DESCRIPTION OF THE DRAWINGS

The invention, together with further objects and advantages thereof, may best be understood by referring to the following detailed description ,taken together with the accompanying drawings, in which:

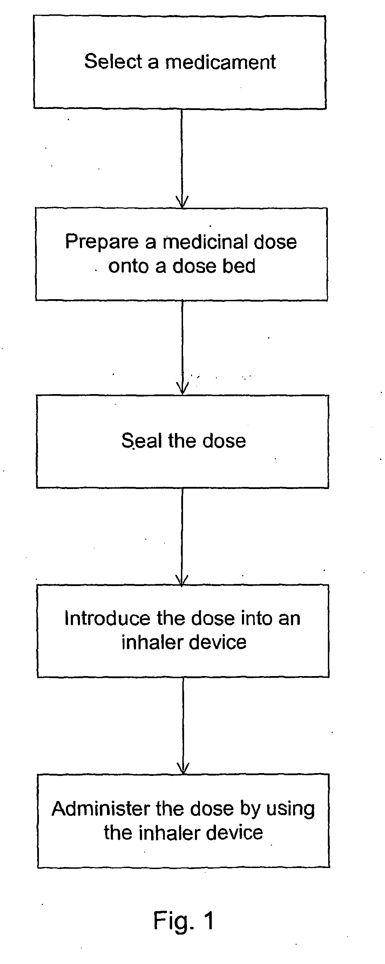

- FIG. 1: illustrates a flow chart over the method of the present invention;

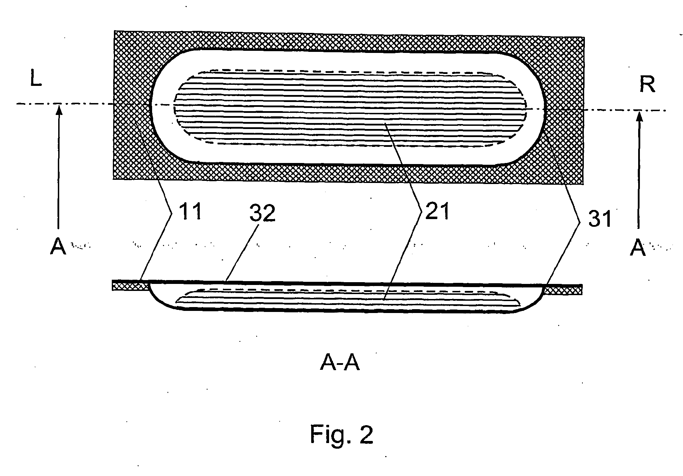

- FIG. 2: illustrates in top and side views a first embodiment of a dose deposited onto a dose bed and a high barrier seal;

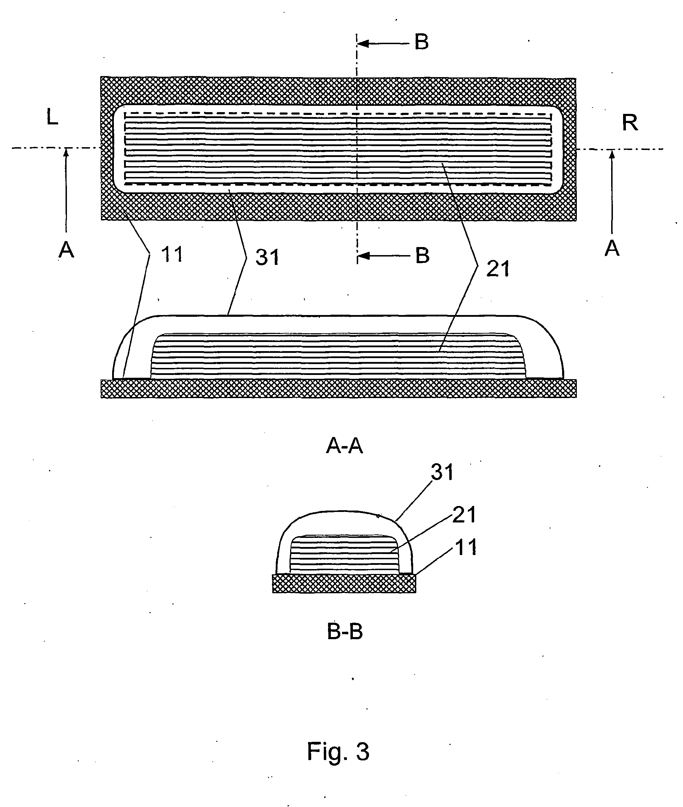

- FIG. 3: illustrates in top and side views a second embodiment of a dose onto a dose bed and a high barrier seal.

### SUMMARY

Metered dry powder medicinal doses are prepared comprising metered deposits of an anticholinergic agent, e.g. oxitropium bromide or preferably ipratropium bromide and most preferably tiotropium bromide in effective quantities, optionally including diluents or other excipients. "Oxitropium", "Ipratropium" and "Tiotropium" refer hereinafter to all the various chemical forms of an active substance, which is suitable for an intended therapeutic effect and in particular to a bromide salt. Because of the potency of the respective drugs it may be necessary to dilute the active substances using a pharmacologically acceptable diluent or excipient in order to meter a correct amount in the forming of doses.

A user introduces the medicinal dose of an anticholinergic agent, e.g. tiotropium into an inhaler device, which may be adapted for a prolonged delivery of the dose during the course of a single inhalation. By means of an Air-razor device applied in the inhaler the dose is gradually aerolized when delivered to the user. Applying an inhaler for a prolonged delivery and using the Air-razor method on a dose comprising the anticholinergic agent, particularly tiotropium, result in a delivered dose composed of a high proportion of de-aggregated fine particles of the selected medicament, whereby an intended prophylactic, therapeutic and psychological effect on the user is achieved while minimizing adverse side effects.

Furthermore, a pharmaceutical dry powder dose of the anticholinergic agent is disclosed with a dose adapted for inhalation, for the prophylaxis or treatment of a user's respiratory disorder. The pharmaceutical dry powder dose is prepared to comprise a metered deposit of a medicinally effective quantity of the anticholinergic agent, e.g. tiotropium, optionally including diluents or excipients, such that the dose is suitable for being introduced into an adapted inhaler device for a prolonged delivery.

The present method is set forth by the independent claim 1 and the dependent claims 2 to 6, and a pharmaceutical dose is set forth by the independent claim 7 and the dependent claims 8 to 12.

### DETAILED DESCRIPTION

The present invention discloses a new method of administration of an anticholinergic agent, particularly oxitropium bromide or more particularly ipratropium bromide, or most particularly tiotropium bromide for treating respiratory diseases like asthma. "Asthma" is used in this document as a generic term for the different respiratory disorders known in the field of medicine, including the one known as chronic obstructive pulmonary disease, COPD.

An anticholinergic medicament may exist in a pure form of one or more pure active agents, or a medicament may be a compound comprising one or more active agents, optionally formulated together with other substances, e.g. enhancers, carriers, diluents or excipients. Hereinafter, the term "excipient" is used to describe any chemical or biologic substance mixed in with a pure active agent for whatever purpose. In this document, only medicaments in dry powder form are discussed. The term "anticholinergic agent" is in this document a generic term for the respective active chemical substances including pharmaceutically acceptable salts, enantiomers, racemates, hydrates, solvates or mixtures thereof, which have a desired, specific, pharmacologic and therapeutic effect.

A "dose bed" is henceforth defined as a member capable of harboring a metered dose comprising one or more entities of dry powder, where the dose is intended for delivery to a user of a DPI in a single inhalation performed by the user. Different types of pharmaceutical blister packs or capsules are included in the term "dose bed". In a preferred embodiment the dose bed and the deposited dose are sealed moisture-tight in a package adapted for a prolonged delivery, i.e. the delivery period for the doses is in a range from 0.01 to 6 s, usually in a range from 0.1 to 2 seconds, delivery taking place sometime during the course of an inhalation as controlled by a purposefully designed DPI, also adapted for a prolonged delivery of doses. Advantageously, such a DPI adopts an Air-razor method (discussed later) of gradual aerosolization of the doses by introducing a relative motion between an air-sucking nozzle and the powder doses. Advantages of a prolonged delivery of a dose for inhalation are disclosed in our US Patent No. 6,571,793 B1 (WO 02 / 24264 A1), which is hereby incorporated in this document in its entirety as a reference.

In the preferred embodiment the dose is sealed, e.g. by foiling, such that powder of the dose cannot interact in any way with dose bed materials or the seal and so that foreign substances or moisture cannot contaminate the powder. Tiotropium is much more sensitive to moisture than most dry powder medicaments including other anticholinergic agents. It is therefore very important to protect the powder in the dose from water in all forms all the way from the point of manufacture up to the moment of inhalation. Therefore, when selecting tiotropium, e.g. in the form of Spiriva^{®}, as medicament, it must not be contained and stored in a capsule or blister, which lacks high barrier seal protection against ingress of moisture. If moisture can access the powder, the respirable dose with particles less than 5 µm will be only a small share of the metered dose and the fine particle dose (FPD) will become less and less over time.

"High barrier seal" means a water-free packaging construction or material or combinations of materials. A high barrier seal may for instance be made up of one or more layers of materials i.e. technical polymers, aluminum or other metals, glass, siliconoxides, that together constitutes the high barrier seal.

A dose bed carrier is normally engaged to carry at least one dose bed loaded with a dose, whereby the dose bed carrier may be inserted into a DPI for administering the thereby inserted doses, e.g. sequentially, to a user in need of treatment. A suitable dose bed carrier is disclosed in our U.S. Patent No. 6,622,723 B1 (WO 01/34233 A1), which is hereby incorporated in this document in its entirety as a reference. However, a dose bed may be designed to act as a dose bed carrier, intended for direct insertion into a DPI. A suitable DPI for a continuous dose delivery is disclosed in our U.S. Patent No. 6,422,236 B1, which is hereby incorporated in this document in its entirety as a reference.

A method of depositing microgram and milligram quantities of dry powders using electric field technology is disclosed in our US Patent No. 6,592,930 B2, which is hereby incorporated in this document in its entirety as a reference.

Methods of dose forming include conventional mass or volumetric metering and devices and machine equipment well known to the pharmaceutical industry for filling blister packs, for example. Also see European Patent No. EP 0319131B1 and U.S. Patent No. 5,187,921 for examples of prior art in volumetric and/or mass methods and devices for producing doses of medicaments in powder form. Electrostatic forming methods may also be used, for example as disclosed in U.S. Patent Nos. 6,007,630 and 5,699,649. Any suitable method capable of producing metered microgram and milligram quantities of dry powder medicaments may be used. Dose deposits may hold together in a more or less porous entity by action of van der Waals forces, electrostatic forces, electric forces, capillary forces etc interacting between particles and particle aggregates and the dose bed material.

Total mass in doses according to the present invention is typically in a range from 5 µg to 5 mg, but may extend to 50 mg. Regardless of which forming and filling method is being used for a particular medicament, it is important during dose forming to make sure that a selected medicament is metered and deposited onto a target area or into a compartment of the dose bed. The shape of the compartment is governed by physical constraints defined by the type of dose bed used. As an example, a preferred type of dose bed is an elongated strip of a biologically acceptable, inert material, e.g. plastic or metal or combinations thereof, between 5 and 50 mm long and between 1 and 10 mm wide. The strip may further be divided into separate target areas or compartments arranged along the length of the elongated strip. Yet another type of dose bed may be a small bowl or pod, usually made of aluminum or a polymer laminated with a metal foil. The dose bed receives an individual seal, for instance in the form of an aluminum foil, to protect the dose against ingress of moisture and other foreign matter, , in a step immediately subsequent to the dose forming.

**Table 1. Typical dosages of some anticholinergic agents respectively in asthma therapy**

| Medicament active agent | Delivered dosage range per dose (µg) | Delivered dosage range per day for adults (µg) |
|---|---|---|
| Oxitropium bromide | 100-400 | 100-2000 |
| Ipratropium bromide | 1-100 | 40-400 |
| Tiotropium bromide | 1-40 | 1-100 |

A dose is intended for administration in a single inhalation, either irregularly when need arises, or more typically as part of a daily management regime. The number of doses administered regularly may vary considerably depending on the type of disorder. Optimal dosages of an anticholinergic agent for prevention or treatment of respiratory disorders may be determined by those skilled in the art, and will vary with medicament potency and the advancement of the disease condition. Furthermore, factors associated with the individual undergoing treatment determine correct dosages, such as age, weight, sex etc. Depending on what is a correct dosage per day and the number of planned administrations per day, the correct deposit by mass for the selected medicament may be calculated, such that metered deposits constituting a dose may be produced in a dose-forming step. In calculating a correct nominal deposit of mass for a medicament, the fine particle fraction, i.e. particles having a mass median aerodynamic diameter less than 5 µm of the actual delivered dose must be taken into consideration. As discussed in the foregoing, the efficacy of inhalers differs considerably and it is thus important to include the expected efficacy of the chosen inhaler in the calculation of a suitable nominal mass in the deposited entity or entities. What constitutes a suitable amount of the selected anticholinergic agent medicament respectively are indicated in Table 1 above and depend on the factors described in the foregoing. Typically doses according to the present invention, would comprise an inhaled fine particle dose of 65 µg oxitropium bromide or 12 µg ipratropium bromide or 3 µg tiotropium bromide respectively per inhalation.

There is generally a medical need to direct the delivery, i.e. the deposition, of inhaled doses of a medicament to the optimum site of action, where the therapeutic effect is the best possible, in the lung, including the deep lung, either for a topical effect or for a systemic effect. Turning to the case in point, it is of course desirable to control the deposition of the dose of an anticholinergic agent to a preferred site of action in the lung in order to get highest possible overall efficacy for each dose with a minimum of side effects. Aerodynamic particle size is a most important factor greatly influencing where in the airways and lungs particle deposition is likely to take place. From a target site point of view, it is therefore desirable to tailor the physical formulations of the medication powder in the doses in such a way that they result in an advantageous particle aerodynamic size distribution by mass in the delivered dose. The present invention makes it possible to deliver the doses, thus formulated, to the targeted sites of action.

Available data indicate that for best performance, the AD (aerodynamic diameter) for the powders in the delivered doses should be in a range from 1 to 5 µm for a successful deposition in the lung. A dose thus formed may be introduced into a dry powder inhaler (DPI) adapted for a prolonged delivery, such that the medicament entities constituting the dose may be aerosolized and delivered in the inspiration air during the course of a single inhalation by a user.

It is obvious that an inhaler, which instantaneously subjects all powders of the doses to a jet-stream of air will aerosolize the aggregated deposits more or less simultaneously, whereby the medicament powder, still more or less agglomerated, become mixed into the air leaving the mouthpiece. In contrast, an inhaler subjecting the dose to a jet stream gradually, like a moving tornado attacking a corn field beginning in one end and finishing in the other. Thus, the jet stream does not attack all of the powder entities of the doses instantly, but aerosolizes the entities of the doses gradually over time. An object of the invention is to offer better control of dose release and to facilitate a prolonging of dose delivery in order to produce a high fine particle fraction (FPF) in the delivered doses. Another object of the invention is to achieve a high ratio of delivered doses relative metered doses. Although it is possible to successfully apply the invention to prior art inhalers, they tend to deliver the doses more or less mixed in too short a time, resulting in a poor FPF figure and low efficacy. On the other hand, a gradual, well-timed, sequential delivery of a dose is possible using a new inhaler design where a relative movement is introduced between the dose and a suction nozzle through which the inspiration airflow is channeled. This arrangement utilizes the inhalation effort of the user to aerosolize the dose gradually for a prolonged period, thus using the power of the suction more efficiently and eliminating in most cases a need for external power to aerosolize the doses. A method of de-aggregating and dispersing dry medicament powder into air is disclosed in our application US 2003/0192539 A1, which is hereby incorporated in this document in its entirety as a reference.

A powder Air-razor method is advantageously used for aerosolizing the medicament powder entities of the dose, the Air-razor providing de-aggregation and dispersal into air of the finely divided medication powder. The Air-razor concept is described in our Patent application US 2003/0192538 A1, which is hereby incorporated in the document in its entirety as a reference. By utilizing an effort of sucking air through a mouthpiece of an inhaler, said mouthpiece connected to a nozzle, the particles of the deposited medicament powder, made available to the nozzle inlet, are gradually de-aggregated and dispersed into a stream of air entering the nozzle. The gradual de-aggregation and dispersal is produced by the high shearing forces of the streaming air in connection with a relative motion introduced between the nozzle and the powder entities of the .dose. In a preferred embodiment, the medicament powder is deposited onto a dose bed, such that the powder deposits occupy an area of similar or larger size than the area of the nozzle inlet. The nozzle is preferably positioned outside the area of deposits, not accessing the powder by the relative motion until the air stream into the nozzle, created by an applied suction, has passed a threshold flow velocity. Coincidental with the application of the suction or shortly afterwards the relative motion will begin such that the nozzle traverses the powder entities constituting the dose gradually. The high velocity air going into the nozzle inlet provides plenty of shearing stress and inertia energy as the flowing air hits the leading point of the border of the contour of the medicament entities, one after the other. This powder Air-razor method, created by the shearing stress and inertia of the air stream, is so powerful that the particles in the particle aggregates in the powder adjacent to the inlet of the moving nozzle are released, de-aggregated to a very high degree as well as dispersed and subsequently entrained in the created air stream going through the nozzle. If the medicament deposits have been made in a compartment in the dose bed and sealed, then obviously the compartment must be opened up first so that the nozzle can access the deposited powder entities in the compartment when suction is applied. Depending on how the entities are laid out on the dose bed, the nozzle will either suck up the powder entities sequentially or in parallel or in some serial/parallel combination. An arrangement for cutting foil is disclosed in our Swedish patent publication SE 517 227 C2 (WO 02/24266 A1), which is hereby incorporated in this document in its entirety as a reference. The interval between opening of the seal protecting the dose to allow the suction system of the inhaler access to the powder in the dose should be as short as possible to minimize the negative influence of the ambient atmosphere, especially humidity, on the powder. This is of course of special importance when tiotropium is the selected medicament. A preferred embodiment is an inhaler, which opens the seal at the same moment as the suction system accesses the dose. Such an inhaler is disclosed in the previously mentioned publication US 6,422,236 B1.

### Example 1

A comparison of a state-of-the-art system for administration of tiotropium with the present invention combined with a DPI employing an Air-razor device has been compiled using in-vitro data collected in the time period of November 2002 through April 2003. The dry powder medicament was selected to be a commercial sample of Spiriva^{®} (22.5 µg tiotropium bromide monohydrate and 5.50 mg lactose monohydrate) available with the HandiHaler^{®} DPI. The present invention used Spiriva^{®} in bulk form together with the Microdrug C-haler DPI (not commercially available). The results are shown in table 1 below.

**Table 1. Inhaled fine particle dose (FPD) <5 µm in %**

| Calculation based on | Spiriva^{®} in HandiHaler^{®}, commercial sample, FPD | Spiriva^{®} in C-haler, FPD |
|---|---|---|
| Metered dose | 18 % | 47 % |
| Delivered dose | 36 % | 56 % |

The present invention improves the efficacy of dose delivery, compared to the state-of-the-art inhalers on the market today, by at least a factor of 2 and typically 2.5. This is accomplished by an increase in the fine particle dose (FPD) < 5 µm to more than 30 %, preferably to more than 50 %, of the metered dose, compared to typically less than 20 % for prior art inhalers. The implications of this vast improvement and the use of tiotropium as a bronchodilator are much less adverse reactions in users, even to the point of eliminating the risk of death, which may be due to long term treatment with high dosages of LABAs.

Thus, the quality of asthma medicament delivery is dramatically improved compared to prior art performance, leading to important advances in delivering a majority of fine particles of the asthma medicament dose to the intended target area in the user's lung with very little loss of particles settling in the throat and upper airways. Administering an asthma medicament, preferably tiotropium, according to the present invention has a very positive therapeutic effect from a medical, psychological and social point of view on a user in need of asthma treatment.

## Claims

1. A dose of pharmaceutical dry powder, adapted for administration by inhalation using a dry powder inhaler device (DPI), comprising tiotropium bromide or a, hydrate, or solvate, thereof, wherein the dose of the pharmaceutical dry powder is deposited onto a dose bed and adapted for prolonged delivery, and the dose is sealed moisture-tightly by the use of a high barrier seal comprising aluminium.

2. The dose according to claim 1, **characterized in that** when the dose has been introduced into the inhaler device adapted for a prolonged delivery and suction through the inhaler is applied, the powder of the dose will be aerosolized by means of an Air-razor device, whereby a majority by mass of the delivered dose is deposited in the lung during a single inhalation effort by a user.

3. The dose according to claim 2, **characterized in that** the deposited powder of the dose is aerosolized gradually over a period inside the single inhalation effort by the user.

4. The dose according to claim 1, **characterized in that** excipients are included, in the dose.

## Patentansprüche

1. Dosis eines pharmazeutischen Trockenpulvers, angepasst zur Verabreichung durch Inhalation unter Verwendung einer Trockenpulverinhalationsvorrichtung (dry powder inhaler device, DPI), umfassend Tiotropiumbromid oder ein Hydrat oder Solvat hiervon, wobei die Dosis des pharmazeutischen Trockenpulvers auf einem Dosierbett vorliegt und für verlängerte Abgabe angepasst ist, und die Dosis durch Verwendung einer hochwirksamen Abdichtung bzw. Dichtung, die Aluminium umfasst, feuchtigkeitsdicht abgedichtet ist.

2. Dosis nach Anspruch 1, **dadurch gekennzeichnet, dass** nachdem die Dosis in die Inhalationsvorrichtung, die für eine verlängerte Abgabe angepasst ist, eingeführt wurde, und im Inhalator ein Sog vorliegt, das Pulver der Dosis durch eine Luftklingenvorrichtung ("Air-razor device") in ein Aerosol überführt wird, wodurch ein Hauptmasseanteil der zugeführten Dosis während eines einzelnen Inhalationsvorgangs eines Verwenders in die Lunge eingebracht wird.

3. Dosis nach Anspruch 2, **dadurch gekennzeichnet, dass** das vorliegende Pulver der Dosis langsam über einen Zeitraum während des einzelnen Inhalationsvorgangs des Verwenders in ein Aerosol überführt wird.

4. Dosis nach Anspruch 1, **dadurch gekennzeichnet, dass** in der Dosis Hilfsstoffe enthalten sind.

## Revendications

1. Dose de poudre pharmaceutique sèche, adaptée pour une administration par inhalation au moyen d'un inhalateur de poudre sèche (DPI), comprenant du bromure de tiotropium ou un hydrate ou solvate de celui-ci, dans lequel la dose de poudre pharmaceutique sèche est déposée sur un lit pour dose et est adaptée pour une distribution prolongée, et la dose est protégée hermétiquement contre l'humidité au moyen d'une barrière d'étanchéité élevée comprenant de l'aluminium.

2. Dose selon la revendication 1, **caractérisée en ce que,** quand la dose a été introduite dans l'inhalateur adapté pour une distribution prolongée et qu'une aspiration à travers l'inhalateur est appliquée, la poudre de la dose forme un aérosol au moyen d'un dispositif à rasoir d'air, moyennant quoi une majorité en poids de la dose délivrée est déposée dans les poumons lors d'un unique effort d'inhalation par un utilisateur.

3. Dose selon la revendication 2, **caractérisée en ce que** la poudre déposée de la dose est progressivement transformée en aérosol pendant une période au cours de l'unique effort d'inhalation par l'utilisateur.

4. Dose selon la revendication 1, **caractérisée en ce que** des excipients sont inclus dans la dose.
